# EUROPEAN PATENT SPECIFICATION

(11) **EP 3 330 369 B1**
(45) Date of publication and mention of the grant of the patent: **09.09.2020**
(21) Application number: 16832928.2
(22) Date of filing: 29.07.2016
(51) Int. Cl.: C12N 5/00, A61L 27/00, C12N 5/0775

(54) **CAPILLARY VESSEL-DERIVED STEM CELLS, USE OF THE SAME, AND, METHOD FOR PRODUCING THE SAME**
STAMMZELLEN AUS BLUTKAPILLARE, VERWENDUNG DAVON UND VERFAHREN ZUR HERSTELLUNG DAVON
CELLULES SOUCHES ISSUES D'UN CAPILLAIRE SANGUIN, UTILISATION DE CELLES-CI ET PROCÉDÉ DE PRODUCTION DE CELLES-CI

(30) Priority: 31.07.2015 JP 2015151601
(43) Date of publication of application: 06.06.2018
(73) Proprietor: National University Corporation Asahikawa Medical University, Hokkaido 078-8510 (JP)
(72) Inventor: KAWABE, Junichi, Asahikawa, Hokkaido 0788510 (JP); NAGAOKA, Taiji, Asahikawa, Hokkaido 0788510 (JP); YOSHIDA, Akitoshi, Asahikawa, Hokkaido 0788510 (JP); HASEBE, Naoyuki, Asahikawa, Hokkaido 0788510 (JP)
(74) Representative: Arends, William Gerrit
(86) International application number: PCT/JP2016/072259
(87) International publication number: WO 2017/022649

(56) References cited:
- WO-A1-2013/118786
- US-A1- 2007 264 239
- FENG-JUAN LV ET AL: "Concise Review: The Surface Markers and Identity of Human Mesenchymal Stem Cells", STEM CELLS, vol. 32, no. 6, 23 June 2014 (2014-06-23), pages 1408-1419, XP055152721, ISSN: 1066-5099, DOI: 10.1002/stem.1681
- MASOUD MALEKI ET AL: "Comparison of Mesenchymal Stem Cell Markers in Multiple Human Adult Stem Cells", INTERNATIONAL JOURNAL OF STEM CELLS, vol. 7, no. 2, 30 November 2014 (2014-11-30), pages 118-126, XP055408105, ISSN: 2005-3606, DOI: 10.15283/ijsc.2014.7.2.118
- LIU DONG-CAI ET AL: "MTDH and EphA7 are markers for metastasis and poor prognosis of gallbladder adenocarcinoma", DIAGNOSTIC CYTOPATHOLOGY, vol. 41, no. 3, March 2013 (2013-03), pages 199-205, XP002787340, ISSN: 8755-1039, DOI: 10.1002/DC.21821
- CRISAN, MIHAELA et al.: "Perivascular Multipotent Progenitor Cells in Human Organs", Hematopoietic Stem Cells VII, vol. 1176, no. 1, 25 September 2009 (2009-09-25), pages 118-123, XP055091952,
- CHEN, WILLIAM C. W. et al.: "Isolation of blood- vessel-derived multipotent precursors from human skeletal muscle", Journal of Visualized Experiments, vol. 90, 21 August 2014 (2014-08-21), pages 1-6, XP055362847, & DATABASE CAplus American Chemical Society; retrieved from STN Database accession no. 2014:1841245
- JUN'ICHI KAWABE: "Heisei 25 Nendo 'Innovative Research in Life Science ' Project-gata Kenkyu Kadai Research Projects for the Clinical Application using Capillary Stem Cells", Asahikawa Medical University research bulletin, vol. 15, no. 1, February 2015 (2015-02), pages 94-98, XP009509044,
- MAKI KABARA ET AL.: "P-02-034: Mosai Kekkan Shusaibo kara no Shinki no Tabunkano Saibo (Capillary Stem Cells; CapSCs) no Dotei to sono Kino Kaiseki", THE JAPANESE SOCIETY FOR REGENERATIVE MEDICINE ZASSHI REGENERATIVE MEDICINE EXTRA ISSUE, vol. 14, 1 February 2015 (2015-02-01), page 328, XP009508941, ISSN: 1347-7919

## Description

### Technical Field

### [Related Application]

This description includes the contents as disclosed in the specification of Japanese Patent Application No. 2015-151601 (filed on July 31, 2015), which is a priority document of the present application.

### [Technical Field]

The present invention relates to a human-derived mesenchymal stem cell-like cell population expressing a specific surface marker, and medical materials (pharmaceutical compositions, medical devices, and medical products) for tissue or organ regeneration, comprising the cell population.

### Background Art

Tissue stem cells such as mesenchymal stem cells (MSCs) are present in adult tissue and deeply involved in maintenance of the structures and functions of the organ and tissue, and tissue regeneration and remodeling under pathological conditions of various diseases. In recent years, mesenchymal stem cells are expected to be applied to regenerative medicine including cell introduction methods actively using the regenerative capacity thereof.

Pericytes (PCs) are involved in stabilization of vascular structures, as well as functions such as permeability and contractility of vascular walls. Recently, among capillary pericytes within various tissues, cells having mesenchymal stem cell-like multipotency (multipotent pericyte) have been reported one after another.

Because of knowledges (1) that multipotent pericytes are cells of vascular structures distributed throughout *in vivo* tissues, and (2) that a special environment within tissue, in which multipotent pericytes can be maintained as stem cells, is present in perivascular locations (Vascular Niche), multipotent pericytes are particularly receiving attention as basic stem cells involved in tissue regeneration and remodeling such as fibrosis, among tissue stem cells.

Establishment of a method for separating multipotent pericytes from normal human tissues is essential for characterization multipotent pericytes, and further for clinical application using (or targeting) the cells. Pericytes are identified with general pericyte markers (NG2, PDGFRβ, CD146), but no method has existed for selectively identifying multipotent pericytes having multipotency (Non Patent Literature 1). Recently, it has been reported that Nestin- or β3Tubulin-expressing pericytes are pericytes having multipotency (Non Patent Literatures 2, 3). However, these markers are all internally localized in cells, and thus a method for separating multipotent pericytes as living cells from not only human tissues, but also from experimental animal tissues has not been established yet, except for the use of a genetically modified animal that emits fluorescence in association with the expression of the modified gene.

The present inventors have established 10 lineages of clone pericyte cell lines derived from peripheral tissue capillaries using temperature-sensitive SV40T antigen-expressing mice, and then discovered a plurality of multipotent pericyte-cell lines from these cell lines (Patent Literature 1). The clone cells are neural stem cell (NSCs)-like cells having differentiation potency into neural cells in addition to mesenchymal stem cell-like multipotency as conventionally reported, and have been confirmed to retain excellent tissue regenerative capacity such that the cells can differentiate themselves into endothelia or pericytes, so as to construct functional blood vessels essential for tissue regeneration (Non Patent Literature 1).

### Citation List

### Patent Literature

Patent Literature 1: WO2013/118786

### Non Patent Literature

Non Patent Literature 1: Armulik A. et al., Dev Cell. 2011 Aug 16; 21(2):193-215.
Non Patent Literature 2: Birbrair A. et al., Stem Cell Res. 2013 Jan; 10(1):67-84.
Non Patent Literature 3: Stapor PC1, Murfee WL., Microvasc Res. 2012 Mar; 83(2): 257-62.
Non Patent Literature 4: Kabara M, Kawabe J, et al. Lab Invest. 2014; 94: 1340-1354.

### Summary of Invention

### Technical Problem

Objects of the present invention are to specify cell surface markers for selective identification of human peristem cells having vasculogenic potential and functions as tissue stem cells that supply tissue parenchymal cells, conveniently separate multipotent pericytes from normal human tissues using the markers, and apply the cells to tissue regeneration.

### Solution to Problem

To achieve the above objects, the present inventors have selected 3 types of multipotent pericyte-cell lines differing in the degree of differentiation potency from 10 lineages of clone pericyte cell lines (as described above) derived from mouse peripheral tissue capillaries, and conducted comprehensive array comparative analysis on the expressed genes. These cell lines have been cloned from single cells, so as to have uniform genetic backgrounds, and thus the cellular functions (multipotency) of each cell line have been stably retained over a dozen passages. Therefore, gene clusters relating to cellular functions such as differentiation potency can be efficiently extracted through the comprehensive array comparative analysis of the expressed genes.

Hence, the present inventors have discovered EphA7 as a marker, with which multipotent pericytes can be identified and separated, from candidate factors located on cell surface, and have succeeded in isolation of human multipotent pericytes using the marker.

Specifically, the present invention relates to the following (1) to (13).
(1) An isolated mesenchymal stem cell-like human multipotent stem cell population, which is positive for EphA7 and one or more pericyte markers, preferably one or more pericyte markers selected from NG2, PDGFRβ, CD13, CD146, NGFR, α-smooth muscle actin, Desmin, and RGS5, and more preferably NG2.
(2) The cell population according to (1) above, which is positive for one or more neural stem cell markers, preferably one or more neural stem cell markers selected from Nestin, β3Tubulin, S100A, RC2, Sox2, CD133, and FABP7, and more preferably one or more neural stem cell markers selected from Nestin, β3Tubulin, and S100A.
(3) The cell population according to any one of (1) to (2) above, which is negative for CD34 and CD45.
(4) The cell population according to any one of (1) to (3) above, which is not derived from brain.
(5) The cell population according to any one of (1) to (4) above, which is derived from a tissue comprising capillary vessels.
(6) The cell population according to any one of (1) to (5) above, having sphere-forming ability.
(7) The cell population according to any one of (1) to (6) above, which can differentiate into mesenchymal cells. 8334365-1-GLUCAS
(8) The cell population according to any one of (1) to (6) above, which can differentiate into neural cells.
(9) The cell population according to any one of (1) to (8) above, having vasculogenic potential.
(10) A medical material (examples of the medical material include pharmaceutical compositions, medical devices, and medical products) for tissue or organ regeneration, comprising the cell population according to any one of (1) to (9) above.
(11) The medical material according to (10) above, wherein a tissue or an organ is selected from mesenchymal tissues or organs (e.g., bone, cartilage, tendon, fat, blood vessel, skeletal muscle, and cardiac muscle) and neural tissues or organs.
(12) A method for producing a multipotent stem cell population, comprising the steps of:
   separating adherent cells from the cells of the tissue comprising capillary vessels;
   separating cells positive for pericyte markers, preferably one or more pericyte markers selected from NG2, PDGFRB, and CD146, and more preferably NG2, from the adherent cells; and
   separating an EphA7-positive cells from the pericyte marker-positive cells.
(13) The method according to (12) above, wherein the tissue comprising capillary vessels is any one tissue selected from subcutaneous adipose tissue, skeletal muscle tissue, cardiac tissue, renal tissue, and visceral adipose tissue.

### Advantageous Effects of Invention

The cell population of the present invention is a cell population of basic tissue stem cells existing *in vivo* and having purity sufficient for tissue regeneration compared with mesenchymal stem cells isolated using conventionally known cell surface markers. Hence, the cell population has a low risk of malignant transformation, and a low risk of ethical problems or rejections since cells derived from patients can be used.

The cell population of the present invention has high vasculogenesis·regenerative capacity, constructs a vascular system that is a nutrients·oxygen supply route, and can provide an environment (Vascular Niche) for maintaining stem cell functions. Specifically, the cell population is capable of supplying by itself essential elements for tissue regeneration·maintenance.

The cell population of the present invention has multipotency and carries out vasculogenesis and supplies various tissue parenchymal cells. Specifically, the cell population constructs vessels so as to form a basis for tissue regeneration and serves as tissue stem cells, and differentiates into mesenchymal cells (e.g., fat, bone, cartilage, and skeletal muscle), neural cells (e.g., glial cells and Schwann cells).

The cell population of the present invention is a cell population of rational stem cells for organ regeneration, which can, as stem cells, regenerate tissue parenchymal cells, while widely distributed throughout the body as capillary vessel-composing cells, so as to construct capillary vessels serving as the basis for organ regeneration. Therefore, the cell population is extremely useful as a medicine or a medical material for tissue regeneration in various diseases.

### Brief Description of Drawings

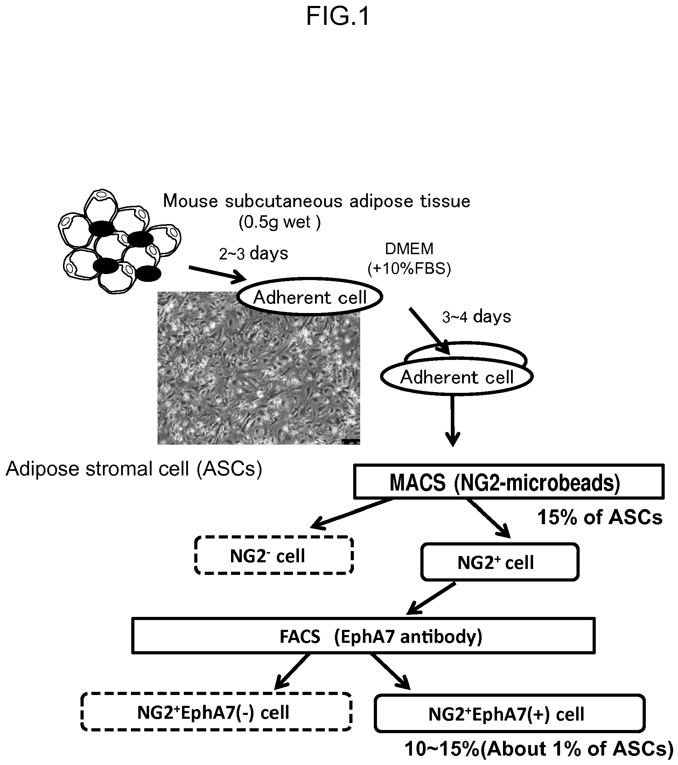

[Figure 1] Figure 1 shows procedures for preparation of mouse capillary stem cells (CapSCs) (NG2-positive EphA7-positive cells).

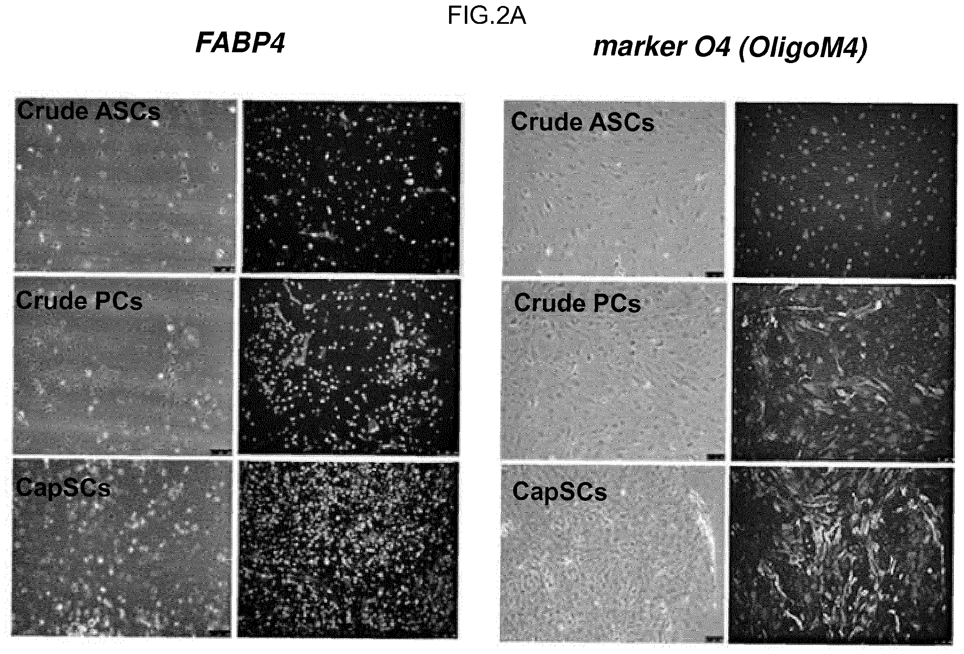

[Figure 2A] Figure 2A shows immunostaining images (left: fat cell differentiation: immunostaining with FABP4, right: neural oligo cell differentiation: immunostaining with marker O4 (OligoM4) (both from top, crude ASCs, crude PCs, and CapSCs)) showing the differentiation of mouse CapSCs (NG2-positive EphA7-positive cells) into fat cells and nerve cells, as compared with crude adipose stromal cells (ASCs) and crude pericytes (NG2-positive EphA7-negative cells).

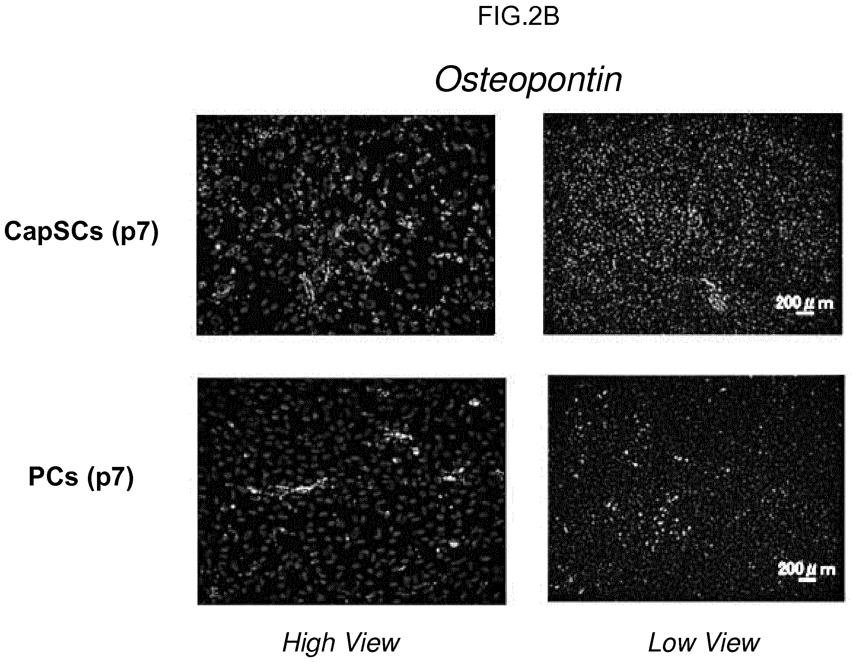

[Figure 2B] Figure 2B shows immunostaining images obtained with osteopontin (upper: CapSCs, lower: PCs (high-power images on the left and low-power images on the right in both cases)) showing the differentiation of mouse CapSCs (NG2-positive EphA7-positive cells) into osteoblasts as compared with PCs (NG2-positive EphA7-negative cells).

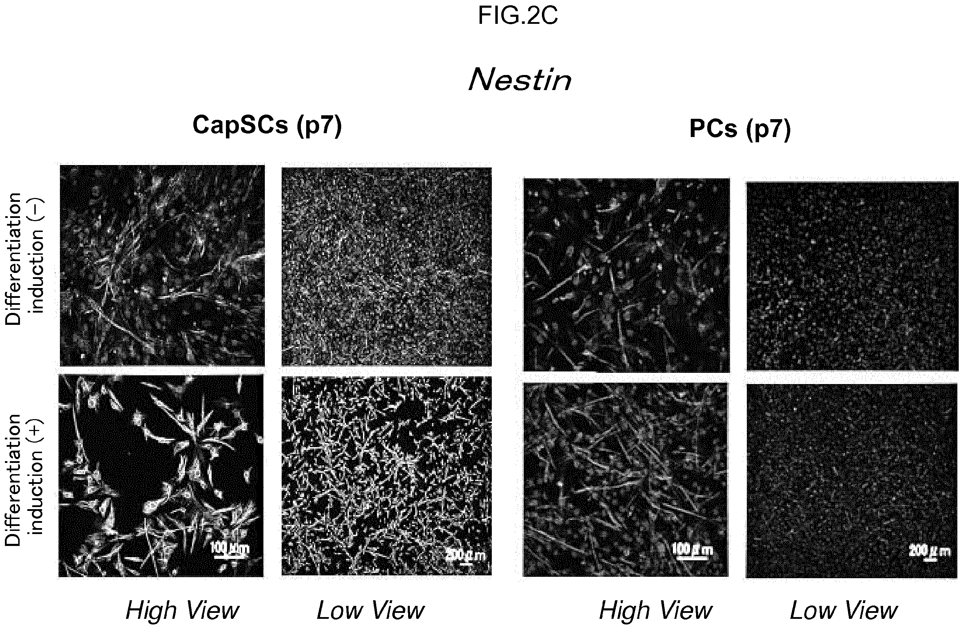

[Figure 2C] Figure 2C shows immunostaining images obtained with Nestin (left: CapSCs, right: PCs (no differentiation induction in both upper images and differentiation induction in both lower images, and high-power images on the left and low-power images on the right in both cases)) showing the differentiation of mouse CapSCs (NG2-positive EphA7-positive cells) into neural stem cells as compared with PCs (NG2-positive EphA7-negative cells).

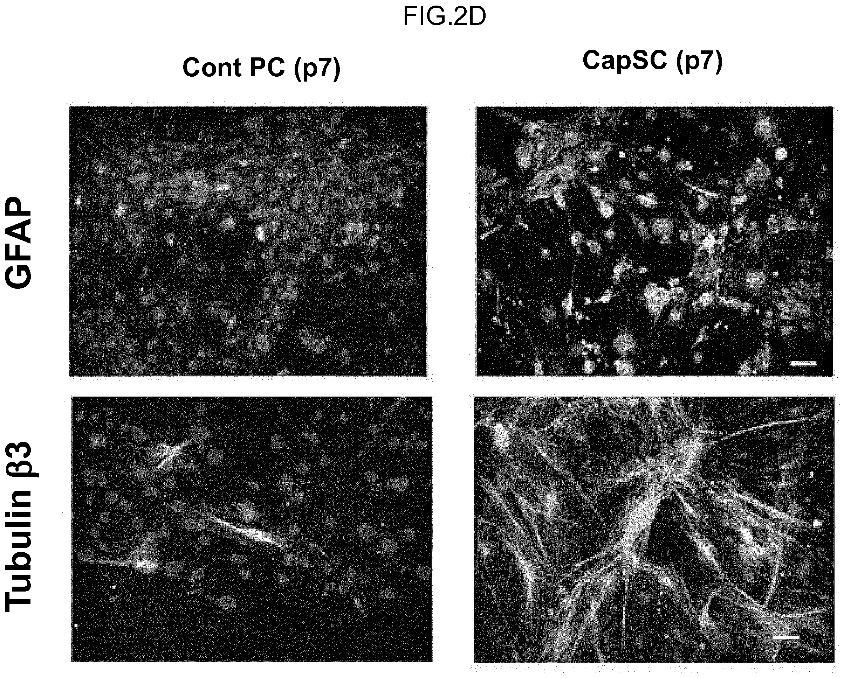

[Figure 2D] Figure 2D shows immunostaining images (left: CapSCs, right: PCs (upper: immunostaining with GFAP, and lower: immunostaining with Tubulinβ3 in both cases)) of the differentiation of mouse CapSCs (NG2-positive EphA7-positive cells) into neural cells (astrocytes and nerve cells) as compared with PCs (NG2-positive EphA7-negative cells).

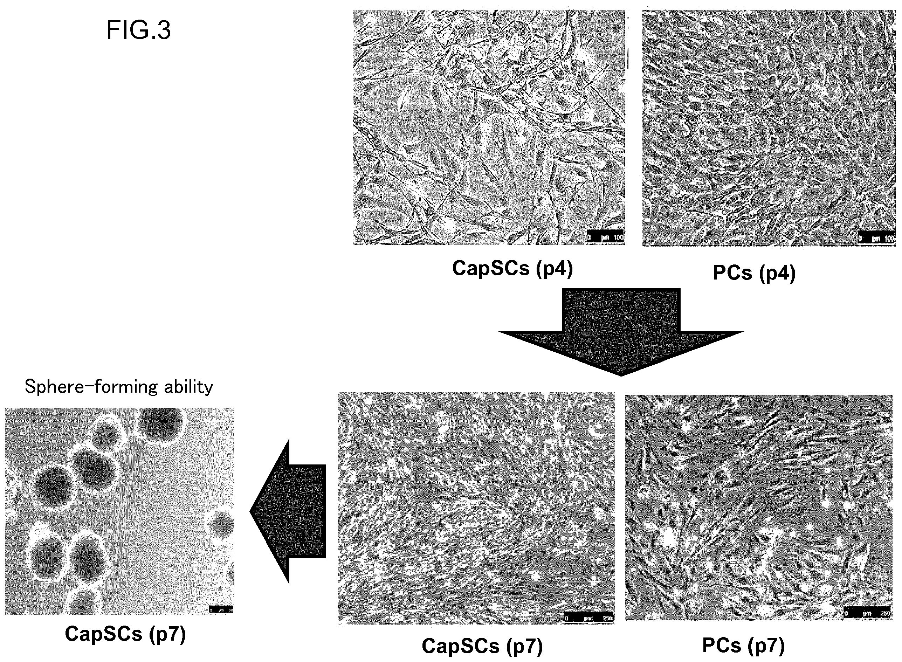

[Figure 3] Figure 3 shows the proliferation potential of mouse CapSCs as compared with PCs (upper: passage 4 (p4), lower right: passage 7 (p7), lower left: sphere formation by CapSCs). Mouse CapSCs had proliferation potential higher than that of PCs, and formed spheres after 7 passages.

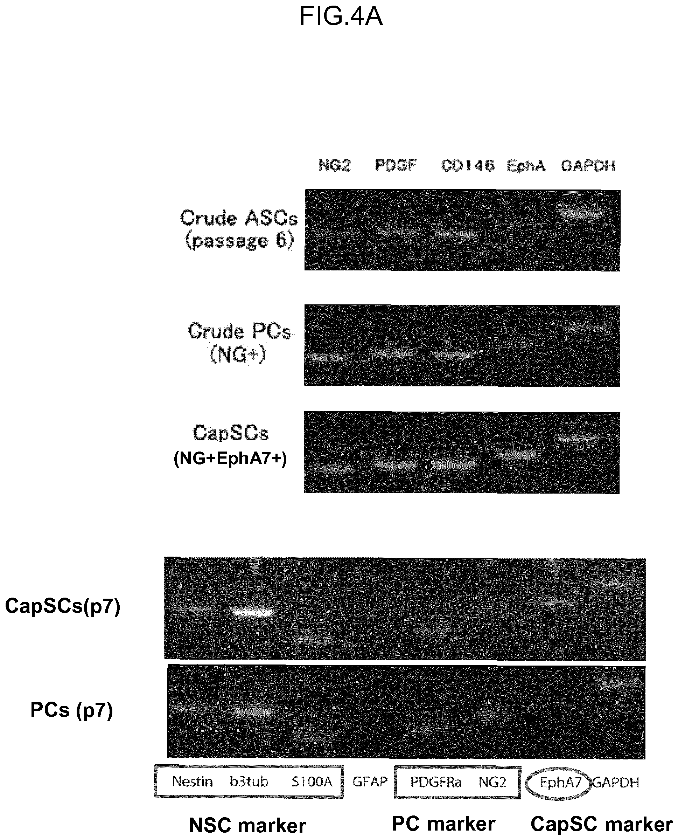

[Figure 4A] Figure 4A shows the results of analyzing by quantitative RT-PCR the gene expression profile of mouse CapSCs as compared with PCs and ASCs. Mouse CapSCs could not be distinguished from PCs and ASCs with known PC markers (NG2, PDGFRβ, CD146) and NSC markers (Nestin, β3Tubulin, S100A), and could be distinguished from the same only with EphA7.

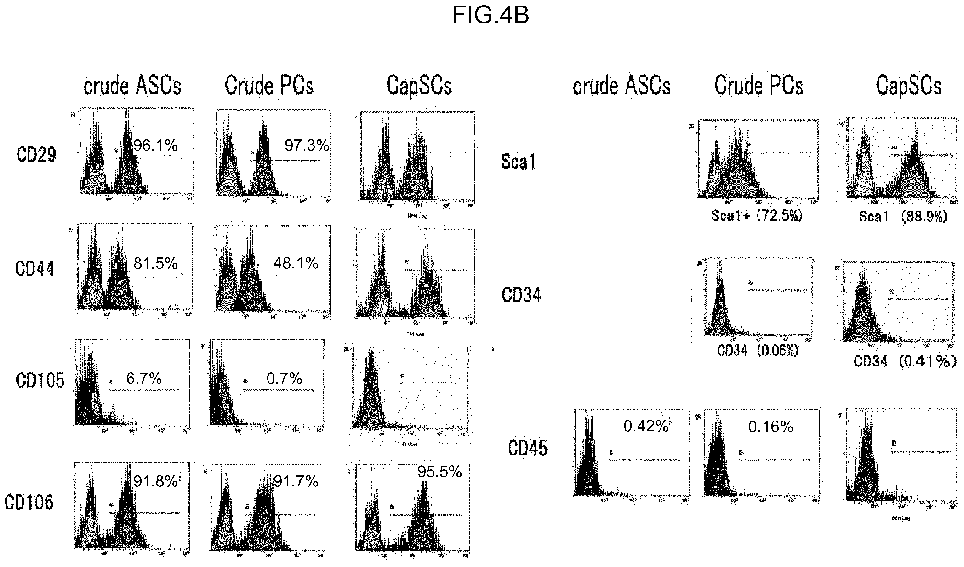

[Figure 4B] Figure 4B shows the results of analyzing the surface marker expression profile of mouse CapSCs by 2 color flow cytometry. Mouse CapSCs were positive for known MSC markers (CD29, 44, 106, Sca1), negative for hematopoietic stem cells (HSC) marker (CD34), and negative for bone marrow·hematopoietic marker (CD45).

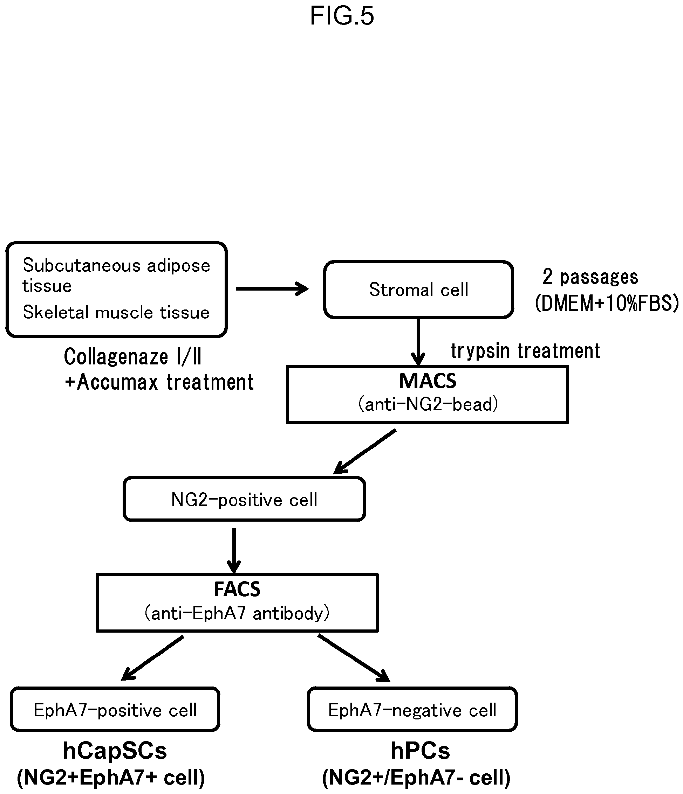

[Figure 5] Figure 5 shows the procedures for preparation of human CapSCs (NG2-positive EphA7-positive cells).

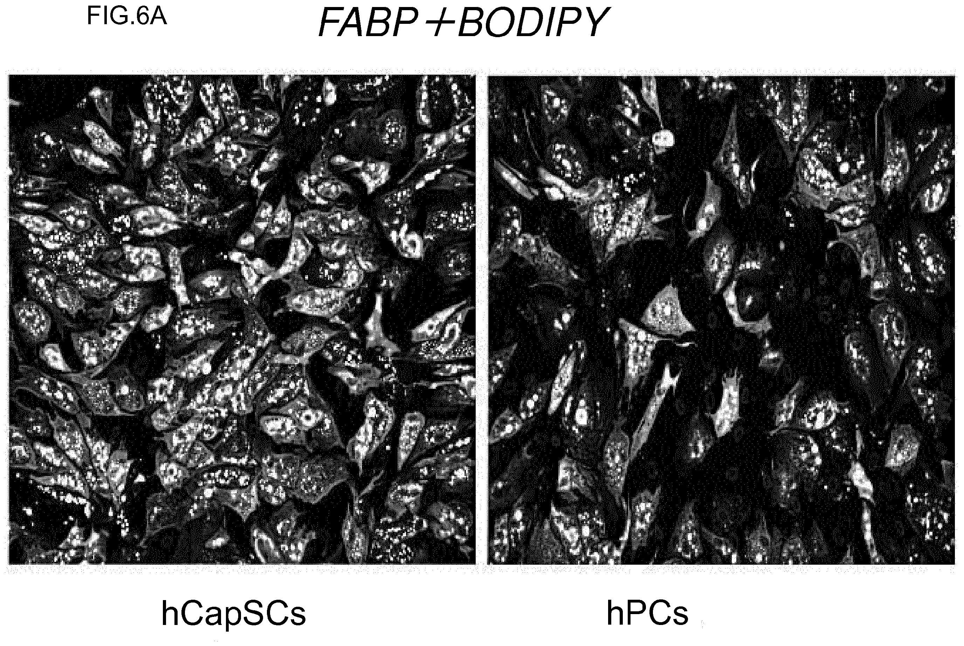

[Figure 6A] Figure 6A shows an immunostaining image (left: human CapSCs, right: human PCs) obtained with FABP (red) showing the differentiation of human CapSCs (NG2-positive EphA7-positive cells) into fat cells and with BODIPY (green) showing the accumulation of lipid droplets.

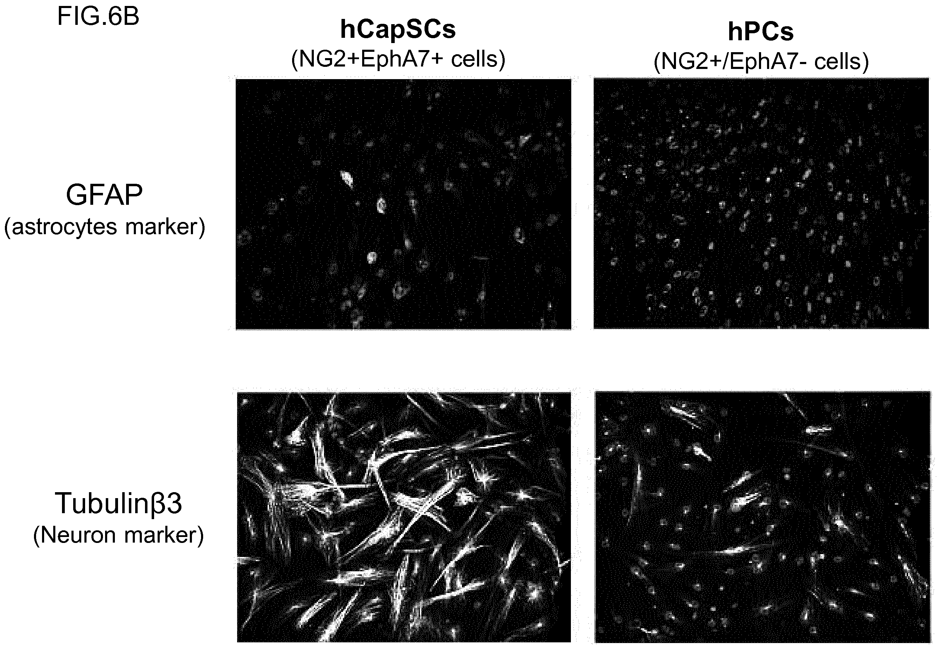

[Figure 6B] Figure 6B shows immunostaining images (left: human CapSCs, right: human PCs) obtained with GFAP (upper) and Tubulinβ3 (lower) showing the differentiation of human CapSCs (NG2-positive EphA7-positive cells) into neural cells.

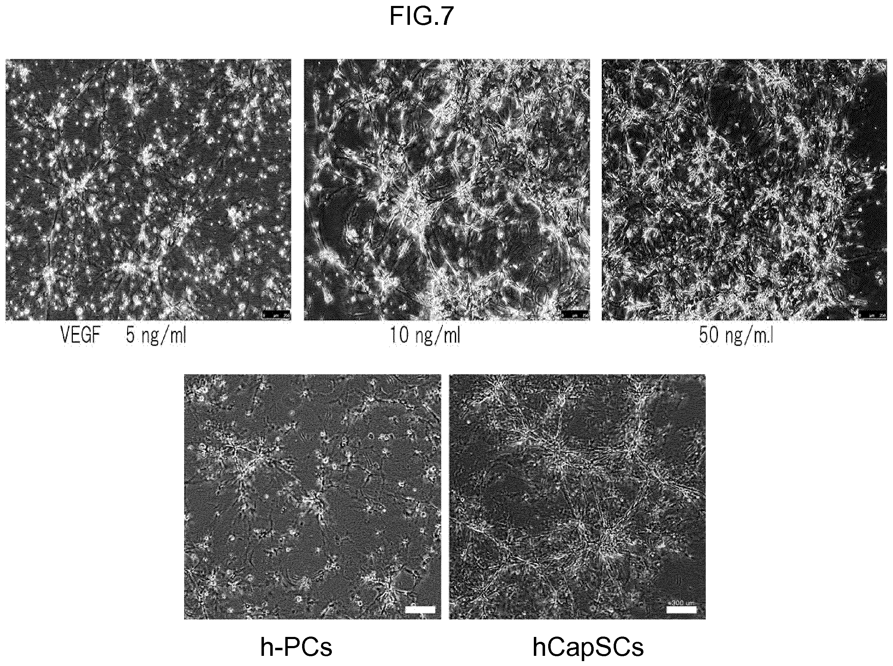

[Figure 7] Figure 7 shows capillary vasculogenesis of human CapSCs on 3-dimensional gel (upper: from the left, VEGF 5 ng/ml, 10 ng/ml, and 50 ng/ml, lower: human PCs on the left, and human CapSCs on the right).

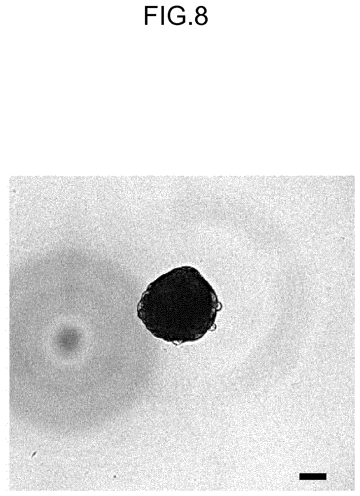

[Figure 8] Figure 8 shows the sphere formation by human CapSCs.

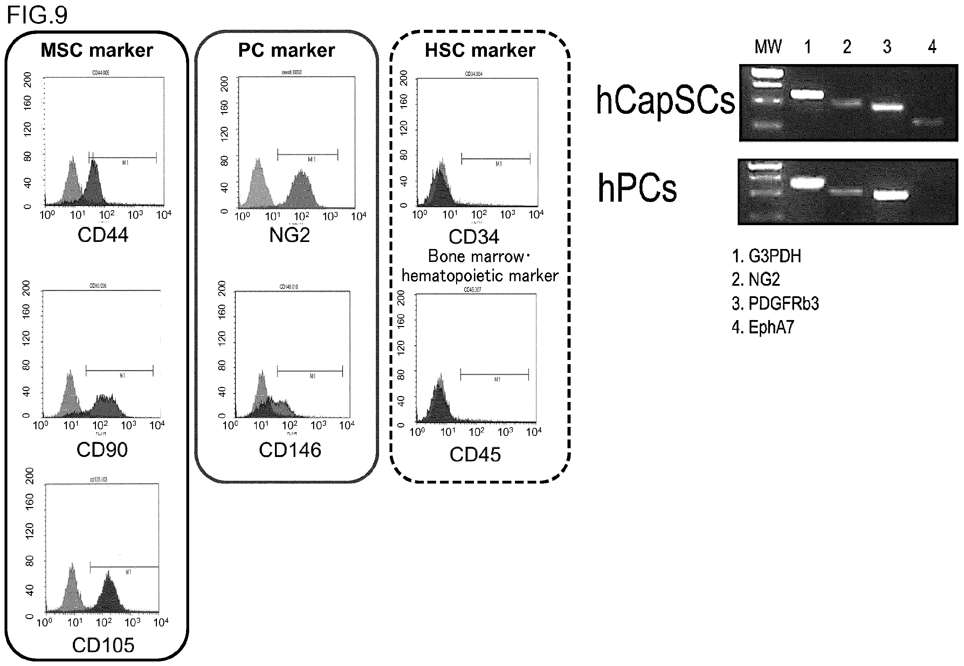

[Figure 9] Figure 9 shows the results of analyzing the surface marker expression profile of human CapSCs by 2 color flow cytometry (left) and quantitative RT-PCR (right). Human CapSCs were positive for known MSC markers (CD44, 90, 105), positive for known PC markers (NG2, CD146) and negative for the HSC marker (CD34) and the bone marrow/hematopoietic marker (CD45). Moreover, human CapSCs could not be distinguished from PCs with known PC markers (NG2, PDGFRβ3), and could be distinguished from PCs only with EphA7.

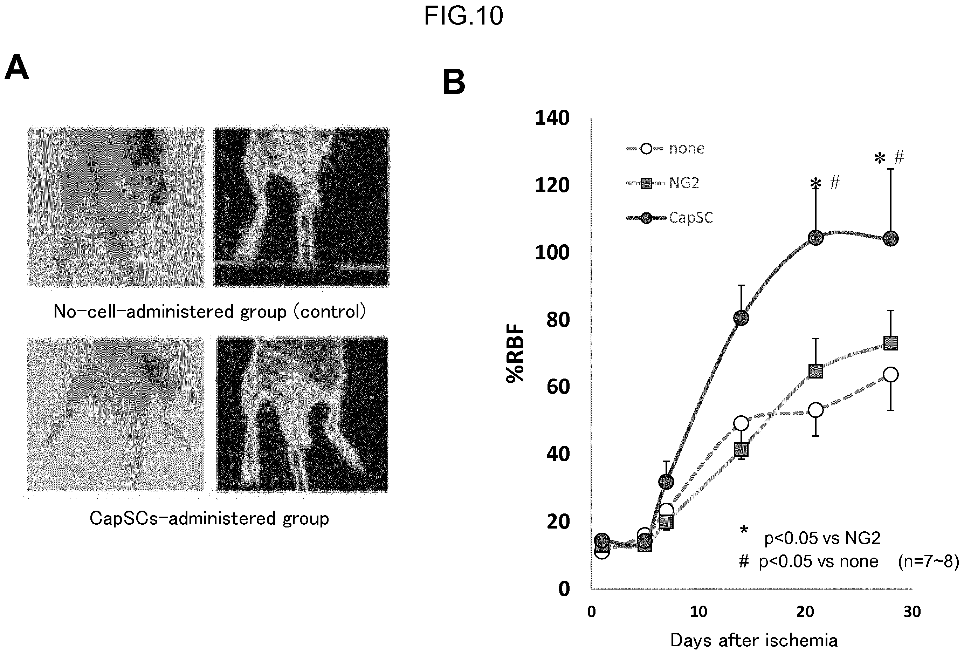

[Figure 10] Figure 10 shows the effects of a therapy for introducing mouse CapSCs into a nude mouse model of severe hindlimb ischemia (A: photographs of ischemic hindlimbs (left) and Doppler measurements thereof (right), B: results of Doppler measurement of blood flow (%RBF)). The CapSCs-administered group was observed to exert significant tissue regeneration (A left) and significant recovery from hind limb ischemia (A right and B), compared with a no-cell-administered group or PCs (NG2-positive EphA7-negative cells) (control).

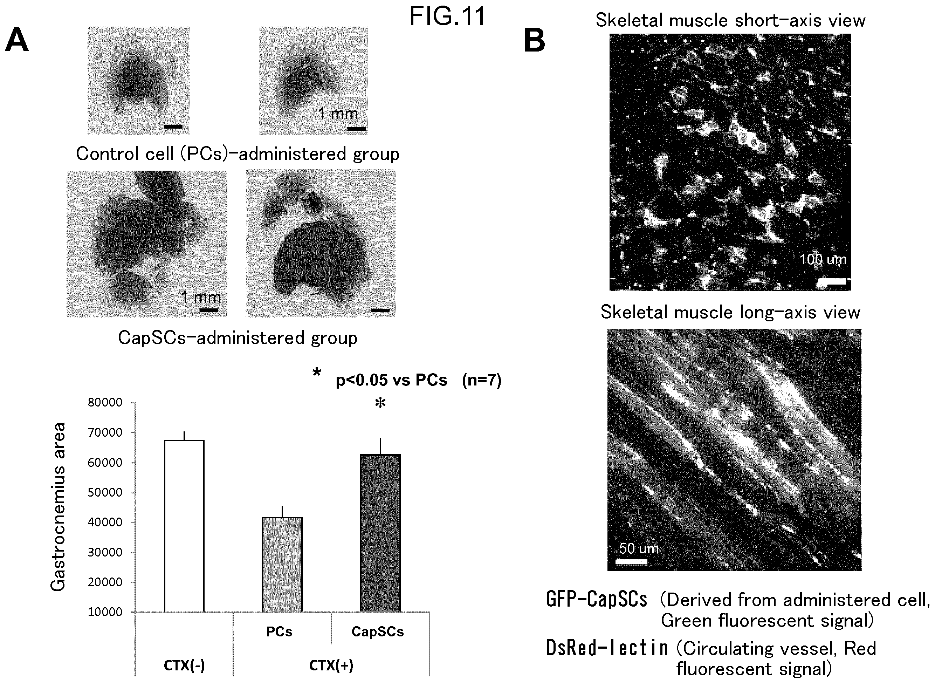

[Figure 11] Figure 11 shows the effect of the administration of mouse CapSCs on damaged gastrocnemius damaged by cardiotoxin (CTX) (A: (upper) HE staining of a short-axis slice of gastrocnemius tissue, (lower) the area of a short-axis slice of gastrocnemius tissue, B: the results of fluorescence observation of short-axis and long-axis slices of gastrocnemius tissue). Compared with control cells (PCs), CapSCs exerted significant effects of improving damaged gastrocnemius, restoring the skeletal muscle mass to a level equivalent to that of non-damaged gastrocnemius (CTX(-)) within 2-3 weeks. Two weeks after introduction of GFP fluorescence-emitting CapSCs, the cells were confirmed to dierentiate into GFP-positive skeletal muscle and blood vessels (red fluorescence staining with lectin) and survive in the tissue.

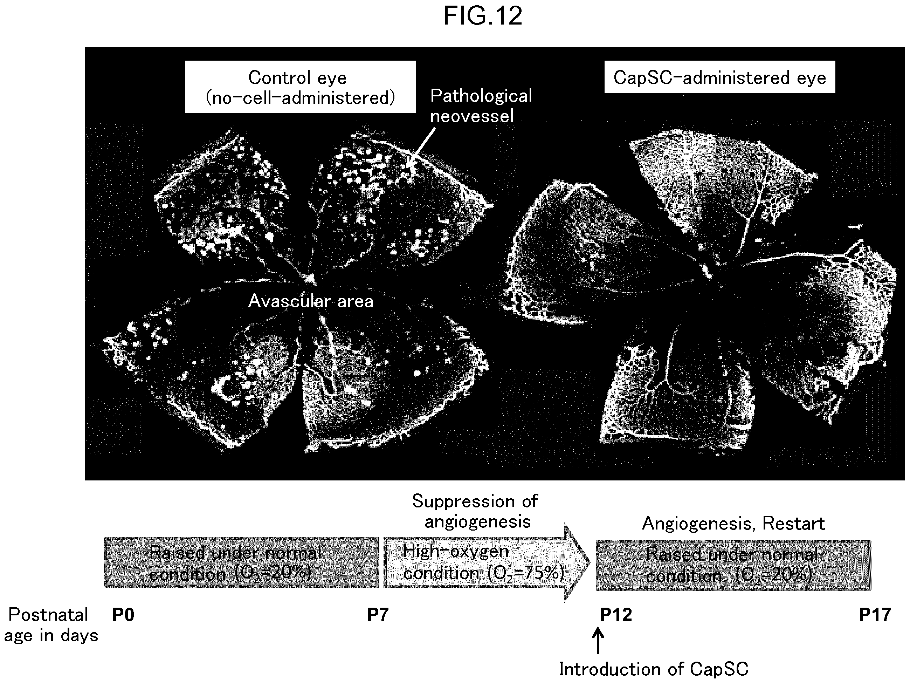

[Figure 12] Figure 12 shows the effect of introduction of mouse CapSCs on a model of oxygen-induced ischemic retinopathy (OIR) (model of retinopathy of prematurity) (left: control eye (to which no cells were administered), right: CapSCs-administered eye). The CapSCs-administered group exerted a decrease in avascular area and improved pathological neovasculogenesis.

### Description of Embodiments

### 1. Cell population (CapSCs) of the present invention

The present invention relates to an isolated mesenchymal stem cell-like multipotent stem cell population characterized by being EphA7 positive. As described later, the cell population of the present invention is isolated from a tissue comprising capillary vessels, so that the "cell population" is also described herein as CapSCs (capillary stem cells).

The term "mesenchymal stem cell-like" as used herein means that such a mesenchymal stem cell-like cell population has properties analogous to those of mesenchymal stem cells. Specifically, the term means that it expresses markers (preferably, 1 or 2 or more markers selected from CD29, CD44, CD90, CD105, CD106 (Sca1 in the case of mouse), and the like) characteristic to mesenchymal stem cells, can differentiate into cells (e.g., fat, bone, cartilage, and skeletal muscle) of tissues belonging to the mesenchymal system, and has properties·morphology peculiar to mesenchymal stem cells.

### (1) Marker

The cell population of the present invention is characterized by being EphA7 positive. "EphA7 (Ephrin type-A receptor 7)" is a protein belonging to an ephrin receptor subfamily of the protein kinase family. Ephrin and ephrin-related receptors are considered to be associated with development, particularly with neural development. Ephrin receptors are classified into two main groups: ephrin-A and ephrin-B based on the extracellular domain sequence and the binding affinity thereof for ligands. "EphA7" is known to be important for cerebral·neural development, the expression of EphA7 in stem cells, particularly in mesenchymal stem cells and pericytes has not been reported. The present inventors have specified that in an established multipotent pericyte-cell line, the "EphA7" is a gene related to multipotency.

The cell population of the present invention expresses markers characteristic to neural stem cells, in addition to markers characteristic to mesenchymal stem cells. Examples of markers characteristic to neural stem cells can include Nestin, Tubulinβ3, S100A, RC2, Sox2, CD133, and FABP7. The cell population of the present invention is positive for 1 or more of these neural stem cell markers and preferably 1 or more markers selected from Nestin, β3Tubulin, and S100A. The cell population of the present invention may further express markers characteristic to pericytes. Examples of markers characteristic to pericytes can include NG2, PDGFRβ, CD13, CD146, NGFR, α-smooth muscle actin, Desmin, and RGS5. The cell population of the present invention is positive for 1 or more of these pericyte markers and preferably NG2.

Meanwhile, the cell population of the present invention is negative for CD34 that is an HSC marker, and is also negative for CD45 that is a bone marrow·hematopoietic marker.

The expression of a marker at the genetic level can be quantitatively confirmed according to a standard method using RT-PCR, DNA chip, and the like. In the case of a surface marker, expression can be conveniently and quantitatively confirmed at the protein level through the use of immunostaining, flow cytometry and the like using a labeled antibody specific to the marker (antigen). In addition, cell separation using surface markers is described in detail in the following section.

### (2) Origin

The cell population of the present invention is a cell population of tissue stem cells that exist throughout the whole human body, and the origin thereof is not particularly limited, but it is preferably not derived from brain. The cell population of the present invention can be efficiently isolated from tissue capillary vessels or a tissue comprising capillary vessels. Examples of the "tissue comprising capillary vessels" can include subcutaneous adipose tissue, skeletal muscle tissue, cardiac tissue, renal tissue, and visceral adipose tissue.

The cell population of the present invention may be a cell population of cells directly isolated from human tissues or cells obtained by subculturing the cells, or a cell line established from these cells. Individual CapSCs contained in the cell population of the present invention exist naturally dispersed throughout the whole-body tissues as described above, and locally exist at density appropriate for the environment, expressing functions appropriate for the microenvironment. In the present invention, as described later, CapSCs are separated using EphA7 that is a specific marker, and then CapSCs are purified·enriched to prepare a cell population, so that the cell population can exert excellent effects which could not have been achieved in the natural state, such as treatment of diseases and tissue regeneration. This is demonstrated by Examples 4 to 6, wherein groups to which the CapSC cell population had been applied exerted evident therapeutic effects compared with control groups in which natural CapSC had existed. Specifically, the cell population of the present invention has properties significantly better than those of cells in the natural state.

### (3) Proliferation potential

The cell population of the present invention has excellent proliferation potential when cultured under conditions appropriate for mesenchymal stem cells as described later, and forms spheres when cultured under conditions where cells do not adhere, such as the use of a non-cell-adhesive culture vessel (hereinafter, referred to as "sphere-forming ability").

### (4) Differentiation potency

The cell population of the present invention is a cell population of "mesenchymal stem cell-like" pluripotent stem cells, can differentiate into mesenchymal cells (e.g., fat, bone, cartilage, and skeletal muscle), can also differentiate into neural cells (e.g., glial cells, and Schwann cells), and furthermore are multipotent stem cells also having vasculogenesis capacity as capillary stem cells. In addition, the term "multipotency (multipotent)" as used herein refers to capability of differentiating into cells of a plurality of limited number of lineages, and does not always refer to capability of differentiating into all 3 germ layers (pluripotency). Differentiation potency can be confirmed based on the above-mentioned Kabara M, Kawabe J, et al. Lab Invest. 2014; 94: 1340-1354, for example.

### (5) Morphology

Cell morphologies range from fusiform to polygon characteristic of mesenchymal stem cells. The cells are adhesive, but exert "sphere-forming ability" characteristic of highly proliferative stem cells when cultured under conditions where no cells adhere, as described above.

The cell population of the present invention has, similarly to mesenchymal stem cells, immunosuppressive capability and accumulation capability of being accumulated in inflamed·tissue-injured sites, as well as vasculogenesis in the ischemic tissues of multipotent pericytes and regenerative capacity for injured tissues. Therefore, the cell population of the present invention can accelerate tissue regeneration and remodeling under pathological conditions of various diseases and can be applied to regenerative medicine for tissues and organs.

### 2. Method for preparing CapSCs

The cell population of the present invention can be prepared by separating an EphA7-positive cells from adherent cells derived from a tissue comprising capillary vessels.

### (1) Cell isolation

First, cells are isolated from a tissue comprising capillary vessels. Examples of the "tissue comprising capillary vessels" can include, as described above, subcutaneous adipose tissue, skeletal muscle tissue, cardiac tissue, renal tissue, and visceral adipose tissue. Cells are isolated according to a standard method by treating these tissues with an enzyme (e.g., collagenase and protease) or a commercially available cell dispersion buffer, and the like.

### (2) Cell culture

Isolated cells are seeded in a medium that is generally used for culturing mesenchymal cells, and then subcultured for 3 to 4 days, and preferably at least 2 or more passages. The thus obtained adherent cells are treated with trypsin or trypsin·EDTA or the like and then separated·collected from the vessel. If desired, pericyte marker-positive cells, such as cells positive for one or more markers selected from NG2, PDGFRB, and CD13, and preferably NG2-positive cells may also be separated from the thus obtained adherent cells. Cell separation methods are as described later.

A medium to be used herein is not particularly limited, as long as it is appropriate for culturing mesenchymal cells. Examples of a standard medium can include MEM, DMEM, BME, DME medium, α-MEM, IMEM, ES medium, DM-160 medium, Fisher medium, F12 medium, WE medium, RPMI medium, StemSpan medium, StemPro medium, and a mixture thereof. Alternatively, a commercially available medium for culturing mesenchymal stem cells may also be used.

To a medium, serum or a serum substitute may also be added. Examples of a serum substitute include Knockout Serum Replacement (KSR: Invitrogen), B-27 supplement, N2-supplement, and albumin (e.g., lipid-rich albumin), and can be used instead of serum as long as it does not impair the purpose of the present invention. The concentration in a medium is determined as appropriate depending on serum or a serum substitute to be used herein. Moreover, to a medium, transferrin, fatty acid, a collagen precursor, a trace element (e.g., zinc and selenium), 2-ME, 3'thiolglycerol and the like may also be added as necessary.

Culture is performed under conditions that are generally employed for culturing mesenchymal cells, for example, conditions of the temperature of 37°C and oxygen concentration of about 20%. As known in the art, cell density in a medium produces an effect on cell properties and differentiation direction. In general, cells are preferably subcultured by passaging cells while exchanging media as appropriate, so that the cell density does not exceed a certain level of density (for example, 5,000 cells/cm²).

### (3) Cell separation

Subsequently, EphA7-positive cells are separated from separated adherent cells, or pericyte marker-positive adherent cells, such as adherent cells positive for one or more markers selected from NG2, PDGFRB, and CD13, and preferably NG2-positive adherent cells. NG2, PDGFRB, CD13, and EphA7 are all cell surface antigens (markers), so that the use of antibodies specific to these antigens makes it possible to conveniently separate cells expressing these surface markers. In other words, the cell population of the present invention is a cell population purified and enriched based on the expression of the above pericyte markers and EphA7. Examples of separation methods using antibodies can include methods using cell sorting beads, magnetic cell sorting, and fluorescent cell sorting.

Cell sorting beads are beads capable of binding an antibody corresponding to a target surface marker to the surface through chemical bonding or physical adsorption. Specifically, beads with an antibody bound thereto are reacted with cells, target surface marker-positive cells are bound to beads, and thus the beads can be recovered by centrifugation, and the like. Alternatively, an antibody (primary antibody) against a surface marker is reacted with cells, beads with a secondary antibody bound thereto are reacted with the cells, and thus target surface marker-positive cells can be collected. Cells bound to beads are eluted using a strainer and the like, and can be collected.

Magnetic cell sorting (for example, MACS (R): Magnetic-activated cell sorting) is a sorting technique that involves capturing specific cells with magnet using magnetic beads, to which an antibody against a surface marker has been bound. This technique is capable of simultaneously treating a large number of cells without the need of any extensive apparatus, but is inferior to FACS (described later) in terms of sorting precision. An automatic magnetic cell sorter for automatically performing MACS is commercially available, and cells can be more conveniently sorted and collected with this sorter.

Fluorescent cell sorting (for example, FACS (R) : Fluorescence activated cell sorting) is a sorting technique that involves flowing cells labeled with a fluorescent antibody in a liquid stream so as to pass through a focus of laser beam, measuring fluorescence emitted by individual cells, measuring the antigen level on the cell surface, and sorting specific cells using a cell sorter. Fluorescent cell sorting requires more cost and time than those required by magnetic cell sorting, but results in low cell loss.

In the present invention, methods for separating cells using each surface marker are not limited to the above methods, and any known method may also be used, and a method may be selected as appropriate depending on the amounts of a sample and cells to be sorted, purposes for analysis and use, and the like.

Cells are cultured preferably at "CPC (Cell Processing Center)" in accordance with GMP standards. "Clinical-grade cells" to be administered to a subject are preferably prepared at a facility specially designed to manipulate cells under aseptic conditions, more specifically, at CPC where cleanliness is ensured by air-conditioning control, chamber pressure control, temperature and humidity control, particle counter, HEPA filter, and the like. Moreover, not only such a CPC facility itself, but also all instruments to be used within CPC, are subjected to validation so that the performance is ensured. Preferably the functions thereof are monitored·recorded at all times, and cell treatment procedures at CPC are all desirably controlled·recorded strictly according to "standard procedures".

### 3. Medical materials (pharmaceutical compositions, medical devices, medical products) for tissue or organ regeneration

The cell population of the present invention is a cell population of rational stem cells for organ regeneration, which can, as stem cells, regenerate tissue parenchymal cells (mesenchymal cells and neural cells), while constructing capillary vessels serving as the basis for organ regeneration. Therefore, the cell population of the present invention can be used as a medical material for tissue or organ regeneration. In addition, examples of the "medical material" of the present invention include all products classified as pharmaceutical products (pharmaceutical compositions), medical devices, and medical products in accordance with country and regional statutes·standards.

The cell population to be used as a medical material may be a cell population of autologous cells or heterologous cells, or an established cell line. When infection, rejection and the like are problems, cells derived from patients, which have been prepared from the patients' tissue, are preferably used.

Tissue subjects or organ subjects are not particularly limited, as long as the cell population of the present invention can differentiate, and examples thereof can include mesenchymal tissues or organs such as bone, cartilage, tendon, fat, blood vessel, skeletal muscle, and cardiac muscle, and neural tissues or organs. Specifically, the cell population of the present invention differentiates into mesenchymal cells, and the cells can be used for skeletal muscle regeneration therapy in muscular dystrophy, blood vessel or skeletal muscle regeneration therapy in severe hind limb ischemia, and cardiac muscle regeneration therapy in myocardial infarct. Moreover, the cell population of the present invention differentiates into neural cells, and thus the cells can be used for vascular and nerve regeneration and vascular stabilization therapy in retinopathy, nerve regeneration therapy in demyelinating diseases (EAE), and therapy for cerebral infarction, and the like. Furthermore, the cell population of the present invention differentiates into vascular cells, and then the cells can be used for all organ regenerations and repairs.

In addition, the term "tissue or organ regeneration" refers to repair or regeneration of damaged tissues or organs, or the functions thereof, and examples thereof include, in the case of neural tissue, neuroprotective action (for example, remyelination of axis cylinder), neurotrophic action (for example, replenishing neuroglia cells), cerebral angiogenic action, and nerve regeneration. Examples thereof include, in the case of brain damage, edema reduction, remyelination of axis cylinder, increased neuroglia cells, angiogenesis, and nerve regeneration, and also include cerebral blood flow recovery, recovery from paralysis, and alleviation of pain or numbness. The effects of regeneration can be confirmed by subjecting damaged tissues to physical examination such as X-ray inspection, CT, MRI, ultrasonic inspection, endoscopy, and biopsy, as well as various tests including hematological test, biochemical test, endocrinological test, motor function test, brain function test, cognitive function test, and the like.

Methods for applying the medical material (pharmaceutical compositions, medical devices, medical products) of the present invention to a living body are not particularly limited, and examples thereof that can be employed herein include, depending on application sites, local transplantation by surgical means, intravenous administration, administration via lumbar puncture, local injection, subcutaneous injection, intradermal administration, intraperitoneal administration, intramuscular administration, intracerebral administration, intraventricular administration, and intravenous administration.

The cell population of the present invention has immunosuppressive capability and accumulation capability of being accumulated in inflamed·tissue-injured sites, characteristic of mesenchymal stem cells. The cell population has vasculogenesis in ischemic tissue containing a tumor site and regenerative capacity for regenerating an injured tissue, and is localized in these sites, so as to repair blood vessels and regenerate the tissue.

The medical material of the present invention may also contain scaffolds and components for supporting cell maintenance·proliferation, and administration to affected parts, and other medically acceptable carriers. Examples of components required for cell maintenance·proliferation include medium components such as a carbon source, a nitrogen source, vitamin, mineral, salts, and various cytokines, or extracellular matrix preparations such as Matrigel™.

Examples of scaffolds and components for supporting administration to affected parts include biodegradable polymers; for example, collagen, polylactic acid, hyaluronic acid, cellulose, and a derivative thereof, as well as a complex composed of two or more types thereof, and aqueous solutions for injection; for example, physiological saline, medium, physiological buffer such as PBS, isotonic solutions containing glucose and other adjuvants (for example, D-sorbitol, D-mannose, D-mannitol, sodium chloride). Examples thereof that can be used in combination further include appropriate solubilizers, such as alcohol, specifically, ethanol and polyalcohol, propylene glycol and polyethylene glycol, and nonionic surfactants, such as polysorbate 80 and HCO-50.

Furthermore, the medical material of the present invention may also contain as necessary, a pharmaceutically acceptable organic solvent, polyvinyl alcohol, polyvinyl pyrrolidone, carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, casein, agar, polyethylene glycol, diglycerol, glycerin, propylene glycol, Vaseline, paraffin, stearyl alcohol, stearic acid, mannitol, sorbitol, lactose, a surfactant acceptable as a medical additive, a buffer agent, an emulsifying agent, a suspending agent, a soothing agent, and a stabilizer and the like.

Actual additives are selected individually or adequately selected in combination from the above examples depending on the morphology of the medical material of the present invention, but examples thereof are not limited thereto. For example, when the medical material is used as an injectable formulation, a purified antibody is dissolved in a solvent, such as physiological saline, buffer solution, glucose solution and the like, and the resultant may also contain an adsorption inhibitor, such as Tween80, Tween20, and gelatin, isotonic agents such as glycerin and D-mannitol, stabilizing agents such as human serum albumin, preservatives such as methyl paraben, and local anesthetics such as benzyl alcohol. Moreover, other medicines and the like accelerating tissue regeneration can also be used in combination.

The shape·morphology of the medical material of the present invention is adequately determined depending on a site or tissue for application. For example, the medical material may be processed into a general cell preparation, as well as into a sheet or disc shape and then used. The medical material may also be used for, in addition to *in vivo* organ·tissue regeneration, *in vitro* organ·tissue regeneration.

Symptoms·diseases that can be targets of the medical material of the present invention are symptoms or diseases that can be treated or improved by the regeneration of ischemic sites, skeletal muscle regeneration, cardiac muscle regeneration, nerve regeneration, and the like.

Regarding regeneration of ischemic sites, examples of target diseases include all diseases that need vascular regeneration or regeneration of ischemic sites, for example, wounds including bedsores·skin ulcer, surgical scar, and refractory peptic ulcer disease, inflammatory diseases including chronic inflammatory bowel diseases such as ulcerative colitis and Crohn's disease, severe limb ischemia, ischemic heart diseases including myocardial infarct·angina pectoris·cardiac failure, cerebral infarction, diabetic neuropathy, and cancer with severe ischemia. In particular, diseases that are difficult to treat with general medicines, including severe chronic hindlimb ischemia (arteriosclerosis obliterans, Buerger's disease), refractory ischemic heart disease, cancer with severe ischemia, diabetic angiopathy including retinopathy, and the like are preferable as target diseases.

Regarding nerve regeneration, examples of target diseases can include all diseases that need nerve regeneration, for example, cerebral ischemia, trauma, brain·spinal cord injury, Huntington's disease, amyotrophic lateral sclerosis, cancer pain, epilepsy, cerebral infarction and sequelae thereof, and Parkinson's disease.

Regarding skeletal muscle regeneration, examples of target diseases can include congenital or progressive muscular dystrophy, congenital or distal myopathy, myotonic dystrophy, myotonia syndrome, mitochondrial disease, periodic paralysis, malignant hyperthermia, and ion channelopathy.

Regarding cardiac muscle regeneration, examples of target diseases can include cardiac failure, myocardial infarct, ischemic heart disease, and dilated cardiomyopathy.

Regarding retinal regeneration, examples of target diseases can include all diseases that need retinal vascular regeneration or retinal nerve regeneration, for example, diabetic retinopathy, glaucoma, age-related macular degeneration, retinal vein occlusion, telangiectasia of fovea centralis, retinopathy of prematurity, and hypertensive retinopathy.

Furthermore, application to organ transplantation-associated graft-versus-host disease (GVHD) and the like can also be expected.

The medical material of the present invention is administered in a therapeutically effective dose. The term "therapeutically effective dose" refers to a dose sufficient for prevention, treatment, alleviation and improvement of target symptoms or diseases. The "therapeutically effective dose" is adequately determined depending on pathological conditions to which the medical material is applied and the degree thereof, the route of administration, the number of doses, and the like. When administered transvenously, 1×10⁴ to 1×10¹⁰, preferably 1×10⁵ to 1×10⁸ cells (may be administered in 1 to multiple doses) can be administered depending on disease types, the degree of disease, and the like. The cells of the present invention possess strong regenerative capacity, and thus even a small dose thereof can exert sufficient regeneration effects, compared with general doses known for conventional mesenchymal stem cells, and the like. As described above, the cells of the present invention can be a universal tool for regenerative medicine as a cellular source for supplying vascular network and tissue parenchymal cells.

### Examples

Hereinafter, the present invention will be described more specifically with reference to Examples, but is not intended to be limited to them.

### Example 1: Array analysis of CapSCs cell line

3 types of cell lines (CapSCs #7 (high degree of differentiation), CapSCs #9 (low degree of differentiation), CapSCs #3 (intermediate)) differing in the degree of differentiation potency were selected from 10 lineages of clone pericyte cell lines derived from peripheral tissue capillaries (see WO2013/118786), which had been established from a temperature-sensitive SV40T antigen-expressing mouse. Comprehensive array comparative analysis was conducted for expressed genes using an RNA expression array (Toray Industries, Inc. 3D-Gene array).

First, gene clusters that had exerted fluctuations in RNA expression level in order of #7-3-9 were extracted. Subsequently, 850 factors, each exerting the rate of change 1.5 or more times the threshold were extracted. The factors were narrowed down to 125 candidate factors in consideration of the results of cluster analysis (gene homology, gene functions and intracellular localization) based on Gene Ontology (GO) and the like and pathway analysis and the like. Furthermore, from these factors, 22 factors were extracted as cell marker candidates (GO analysis) localized on the cell surface. From the 22 factors, EphA7 was finally selected as a factor that is actually expressed specifically by the pericyte cell line (CapSCs #7) with high differentiation potency and that can be recognized by flow cytometry analysis using a specific antibody.

As described above, with the use of 3 types of pericyte cell lines we had established differing in differentiation potency, the specific factor, EphA7, was finally selected through various analyses and wet analyses.

### Example 2: Separation and analysis of mouse CapSCs

### 1. Separation of mouse CapSCs using specific cell marker (Figure 1)

Cells were isolated from mouse subcutaneous adipose tissue (0.5 g wet) by treatment with collagenase I/II and Accumax, and then cultured for 2 to 3 days using DMEM containing 10% FBS. Furthermore, media were exchanged and the cells were cultured for 3 to 4 days, adherent cells (adipose stromal cells: ASC) were collected by trypsin treatment.

NG2-positive cells were separated from the collected cells by magnetic cell sorting (MACS) using microbeads on which anti-NG2 antibody had been immobilized. Furthermore, EphA7-positive cells were separated from the NG2-positive cells by flow cytometry (FACS) using an anti-EphA7 antibody. Finally, NG2-positive EphA7-positive cells accounting for 10% to 15% of adipose stromal cells were obtained. Figure 1 shows steps for preparation of cells.

FACS analysis on surface marker expression demonstrates that crude PCs and CapSCs exerted no difference in the expression of conventional MSCs and PCs markers. It can be understood that CapSCs cannot be sorted with these markers.

### 2. Differentiation potency of mouse CapSCs (Figure 2) (1) Fat cell differentiation potency and nerve cell differentiation potency

The above-prepared NG2-positive EphA7-positive cells (mouse CapSCs) were evaluated for the differentiation potency of differentiation into fat cells and nerve cells.

Potency of differentiation into fat cells was confirmed by using Stem Cell Kits, #SC010 (R&D systems), culturing cells for 7-14 days in αMEM (containing 10% FBS and antibiotic) supplemented with adipogenic supplements (hydrocortisone, isobutylmethilxanthine, indomethacin), performing immunostaining with an anti-FABP-4 antibody, performing Oil Red ○ staining, and then performing fluorescence staining with BODIPY (boron-dipyrromethene).

Differentiation into nerve cells was confirmed by using Human/Mouse/Rat Neural Lineage Functional Identification Kit, #SC028 (R&D systems), culturing cells for 2 days in DMEM (containing N2-MAX Media Supplement and antibiotic) supplemented with maintenance supplements (Recombinant human FGF-basic, recombinant human EGF), culturing cells for 7-10 days in the above DMEM containing differentiation supplements (IGF-1, fetal bovine serum), and performing immunostaining with Nestin, β3Tubulin, marker O4 (OligoM4), or S100 antibody. As a control, crude PCs (NG2-positive EphA7-negative cells) and crude ASCs were similarly cultured and stained.

NG2-positive EphA7-positive cells differentiated into fat cells and nerve cells more efficiently than the control (Figure 2A). It was confirmed by the result that NG2-positive EphA7-positive mouse CapSCs are multipotent cells capable of differentiating into mesenchymal cells and nerve cells beyond germ layers.

### (2) Differentiation potency of osteoblast

NG2-positive EphA7-positive cells (mouse CapSCs) were evaluated for differentiation potency of differentiation into osteoblasts. Stem Cell Kits, #SC010 (R&D systems) were used, cells were cultured for 14-21 days in αMEM (containing 10% FBS and antibiotic) supplemented with osteogenic supplements (ascorbate-phosphate, β-glycerolphosphate, recombinant human BMP-2), immunostaining was performed using an anti-osteopontin antibody, and then alizarin-red staining was performed. As a control, pericytes were similarly cultured and then stained. As a result, mouse CapSCs were confirmed to differentiate into osteopontin-positive osteoblasts more efficiently than the control (Figure 2B).

### (3) Differentiation potency of nerve cell

NG2-positive EphA7-positive cells (mouse CapSCs) were cultured in a manner similar to that in (1) above using Human/Mouse/Rat Neural Lineage Functional Identification Kit #SC028 (R&D systems), and then immunostaining was performed using antibodies against Nestin (Figure 2C), GFAP and sheep IgG AF594 (Figure 2D), β3Tubulin and mouse IgG AF594 (Figure 2D). As a control, pericytes were similarly cultured and then subjected to immunostaining. As a result, mouse CapSCs were confirmed to differentiate into nerve cells more efficiently than the control.

### 3. Proliferation potential·sphere-forming ability of mouse CapSCs (Figure 3)

NG2-positive EphA7-positive cells (mouse CapSCs) were subcultured for 7 passages in DMEM supplemented with FGF (10 ng/ml). Mouse CapSCs were confirmed to proliferate and form spheres more efficiently than the control (Figure 3).

### 4. Gene expression profile of mouse CapSCs (Figure 4)

Mouse CapSCs were analyzed for gene expression profile by quantitative RT-PCR. As controls, crude PCs (NG2-positive EphA7-negative cells) and crude ASCs were similarly analyzed for gene expression profile. As a result, the expression levels of known PC markers (NG2, PDGFRβ, CD146) were the same between mouse CapSCs and the controls, confirming that these cells cannot be distinguished with these markers (Figure 4A, upper).

Also regarding NSC markers (Nestin, β3Tubulin, S100A), no difference was found in expression level of each markers between CapSCs and the Controls other than β3Tubulin that expressed at higher level in CapSCs than pericytes.

EphA7 was expressed specifically only by CapSCs, and mouse CapSCs were confirmed to be characterized by EphA7⁺/β3Tubulin⁺⁺ (Figure 4A lower).

Furthermore, mouse CapSCs were quantitatively analyzed for surface marker expression profile using 2 color flow cytometry. As a result, mouse CapSCs were found to be positive for known MSC markers, CD29, 44, 106, and Sca1, and thus cannot be distinguished from other PCs. Moreover, mouse CapSCs were also confirmed to be negative for an HSC marker, CD34, and also negative for a bone marrow·hematopoietic marker, CD45 (Figure 4B).

### Example 3: Separation and analysis of human CapSCs

### 1. Separation of human CapSCs using specific cell marker (Figure 5)

Human neuroblastoma (1 month-old, female)-derived subcutaneous adipose tissue (0.3 g wet) was treated with collagenase I/II and Accumax, so as to isolate cells, and then the cells were subcultured for 2 passages using DMEM containing 10% FBS. Adherent cells (adipose stromal cells: hAPCs) were collected by trypsin treatment.

NG2-positive cells were separated from the collected cells by magnetic cell sorting (MACS) using microbeads on which an anti-NG2 antibody had been immobilized. Furthermore, EphA7-positive cells were separated from NG2-positive cells by flow cytometry (FACS) using an anti-EphA7 antibody. Finally, NG2-positive EphA7-positive cells accounting for 10% to 15% of adipose stromal cells were obtained. Figure 5 shows the steps for preparation of cells.

### 2. Differentiation potency of human CapSCs (Figure 6) (1) Fat cell differentiation potency

The above-prepared NG2-positive EphA7-positive cells (human CapSCs) were evaluated for potency of differentiation into fat cells. Human/Mouse/Rat Neural Lineage Functional Identification Kit, #SC028 (R&D systems) was used, and cells were cultured for 7-21 days in αMEM (containing 10% FBS and antibiotic) supplemented with adipogenic supplements (hydrocortisone, isobutylmethylxanthine, indomethacin), and then immunostaining was performed using an anti-FABP-4 antibody. As a control, crude hPCs were similarly cultured and then subjected to immunostaining. NG2-positive EphA7-positive cells were confirmed to differentiate into fat cells more efficiently than the control (Figure 6A).

### (2) Nerve cell differentiation potency

NG2-positive EphA7-positive cells (human CapSCs) were evaluated for potency of differentiation into nerve cells. Human/Mouse/Rat Neural Lineage Functional Identification Kit, #SC028 (R&D systems) was used and cells were cultured for 2 days in DMEM (containing N2-MAX Media Supplement and antibiotic) supplemented with maintenance supplements (Recombinant human FGF-basic, recombinant human EGF), cultured for 7-10 days in the above DMEM containing differentiation supplements (IGF-1, fetal bovine serum), and then subjected to immunostaining using Nestin, β3Tubulin, marker O4 (OligoM4), or S100 antibody. As a control, NG2-positive EphA7-negative cells were similarly cultured and then subjected to immunostaining. As a result, human CapSCs were found to differentiate into nerve cells, but the control NG2-positive EphA7-negative cells were found to not differentiate into nerve cells (Figure 6B).

### 3. Vaculogenesis of human CapSCs (Figure 7)

NG2-positive EphA7-positive cells (human CapSCs) were cultured in 3-dimensional gel containing 5 ng/ml, 10 ng/ml, and 50 ng/ml VEGF, respectively, which are shown in microscopic images (Figure 7 upper). As a control, microscopic images of crude hPCs cultured in 3-dimensional gel containing 5 ng/ml VEGF are shown as compared with microscopic images of similarly cultured human CapSCs (Figure 7 lower). It could be confirmed by the results that human CapSCs have excellent vasculogenic potential.

### 4. Proliferation potential·sphere-forming ability of human CapSCs (Figure 8)

NG2-positive EphA7-positive cells (human CapSCs) were subcultured up to 30 passages in DMEM supplemented with FGF (10 ng/ml). Human CapSCs proliferated efficiently and formed spheres from single cells (Figure 8) .

### 5. Surface marker expression profile of human CapSCs (Figure 9)

Human CapSCs were analyzed for surface marker expression profile by 2 color flow cytometry. As a result, the expressions of known PC markers (NG2, CD146), and MSC markers (CD44, CD90, CD105) were confirmed, but the expressions of HSC markers (CD45, CD34) were not observed (Figure 9 left).

The expressions of NG2, PDGFRβ3, and EphA7 were compared by quantitative RT-PCR using hPCs as a control. It was thus confirmed that no difference was found in the expressions of NG2 and PDGFRβ3 between human CapSCs and hPCs, and only EphA7 was specifically expressed in CapSCs (Figure 9 right).

### Example 4: Ischemia improvement·tissue regenerative capacity of mouse CapSCs in severe hind limb ischemia model

A hind limb ischemia model was produced using 12-week-old, male Balb/c nude mice through left femoral arteriovenous ligation·extraction, and 3 days later in each group (n=8), 1×10⁴ mouse CapSCs were locally injected into 5 locations of an ischemic limb. After surgery, lower limb blood flow was evaluated over time (1 to 28 days) by laser Doppler (improvement in ischemic limbs/healthy lateral limb ratio immediately after treatment). Moreover, NG2-positive EphA7-negative cells, and a group to which physiological saline for cell suspension (control) had been administered were similarly evaluated.

Photographs of ischemic limbs 2 weeks later and Doppler measurements thereof (Figure 10A), and the result of comparing the resting skin blood flow (%RBF) among 3 groups (Figure 10B) are shown. The CapSCs-administered group was confirmed to exert significant tissue regeneration and significant recovery from hind limb ischemia, compared with the no-cell-administered group (control). Therefore, human CapSCs were confirmed to have capacity of accelerating tissue regeneration in addition to vasculogenesis, and to be able to significantly improve hind limb ischemia.

### Example 5: Effect of mouse CapSCs to improve damaged gastrocnemius

Cardiotoxin (CTX, Sigma-Aldrich; 100 µl of 0.25 mg/ml in saline per mouse) was injected to the gastrocnemius of 8-10-week-old male SCID mice, so as to damage skeletal muscle (each group: n=7). 4 hours later, 1×10⁵ DsRed-expressing mouse CapSCs were suspended in 200 µl of physiological saline, and then the resultant was injected to damaged gastrocnemius. As a control, NG2-positive EphA7-negative pericytes (PCs) were similarly injected to damaged gastrocnemius. Rhodamine-Lectin was injected via tail vein 2-3 weeks after damage, perfusion fixation was performed using paraformaldehyde, and then gastrocnemius was extracted.

Short-axis slices of gastrocnemius were subjected to hematoxylin-eosin (HE) staining (Figure 11A upper), and then the area of each of the slices was measured to evaluate skeletal muscle mass (Figure 11A lower). CapSCs exerted significant effects of improving damaged gastrocnemius, compared with control cells (PCs), so that within 2-3 weeks, the skeletal muscle mass of damaged gastrocnemius was restored to a level equivalent to that of non-damaged ((-)cardiotoxin) gastrocnemius. Moreover, short-axis and long-axis slices of gastrocnemius tissue were observed by fluorescence imaging (Figure 11B). As a result of fluorescence observation, CapSCs were confirmed to differentiate not only to skeletal muscle cells, but also to capillary vessels, so as to exert excellent skeletal muscle regenerative capacity. In particular, some of CapSCs localize around capillary vessels where CapSCs are originally present, so that CapSCs are retained as tissue stem cells in regenerated tissue, and may partially contribute to the acquisition of the original tissue's own self-repair capacity.

### Example 6: Effect of mouse CapSCs to improve retinopathy

An oxygen-induced retinopathy (OIR) model was used as a retinopathy model. Seven-day-old C57BL/6J mice were exposed to 75% oxygen for 5 days, so that widespread retinal non-perfusion areas were formed in 12-day-old mice. Thereafter, the 75%-oxygen environment was returned to an environment with normal oxygen concentration, and the mice were raised for 5 days, so that pathological retinal neovessels were developed by 17 days old. In this study, 1×10⁴ mouse CapSCs were administered to only one eye (administered eye) of the same 12-day-old OIR mouse, and the other eye was designated as a control eye (=no cell administered) to which no cells had been administered. Rhodamine-Lectin was injected via tail vein in the 17-day-old mouse to visualize circulating blood vessels, eyes were extracted, whole mount specimens were prepared, and then the effects of CapSCs on pathological retinal vasculogenesis were examined.

As shown in fluorescence microscopic observation in Figure 12, whereas hump-shaped pathological neovessels were observed widespread in the control eye, pathological retinal neovessels were remarkably suppressed and normal retinal vascular structure was re-constructed in the administered eye. Therefore, mouse CapSCs were confirmed to accelerate the regeneration of normal retinal vessels and significantly suppress retinopathy.

### Industrial Applicability

The cell population of the present invention is a cell population of rational stem cells for organ regeneration, which can, as stem cells, regenerate tissue parenchymal cells, while constructing capillary vessels serving as the basis for organ regeneration. Therefore, the cell population is extremely useful as a medicine for tissue regeneration in various diseases.

## Claims

1. An isolated mesenchymal stem cell-like human multipotent stem cell population, which is positive for EphA7 and one or more pericyte markers.

2. The cell population according to claim 1, which is positive for one or more neural stem cell markers.

3. The cell population according to claim 1 or 2, which is negative for CD34 and CD45.

4. The cell population according to any one of claims 1 to 3, which is not derived from brain.

5. The cell population according to any one of claims 1 to 4, which is derived from a tissue comprising capillary vessels.

6. The cell population according to any one of claims 1 to 5, having sphere-forming ability.

7. The cell population according to any one of claims 1 to 6, which can differentiate into mesenchymal cells.

8. The cell population according to any one of claims 1 to 6, which can differentiate into neural cells.

9. The cell population according to any one of claims 1 to 8, having vasculogenic potential.

10. A medical material for tissue or organ regeneration, comprising the cell population according to any one of claims 1 to 9.

11. The medical material according to claim 10, wherein a tissue or an organ is selected from mesenchymal and neural tissues or organs.

12. A method for producing a multipotent stem cell population, comprising the steps of:
separating adherent cells from the cells of the tissue comprising capillary vessels;
separating pericyte marker-positive cells from the adherent cells; and
separating an EphA7-positive cells from the pericyte marker-positive cells.

13. The method according to claim 12, wherein the tissue comprising capillary vessels is any one tissue selected from subcutaneous adipose tissue, skeletal muscle tissue, cardiac tissue, renal tissue, and visceral adipose tissue.

## Patentansprüche

1. Isolierte mesenchymale stammzellähnliche humane multipotente Stammzellpopulation, die für EphA7 positiv ist, sowie ein oder mehrere Perizytenmarker.

2. Zellpopulation nach Anspruch 1, die für einen oder mehrere neurale Stammzellmarker positiv ist.

3. Zellpopulation nach Anspruch 1 oder 2, die für CD34 und CD45 negativ ist.

4. Zellpopulation nach einem der Ansprüche 1 bis 3, die nicht himabstammend ist.

5. Zellpopulation nach einem der Ansprüche 1 bis 4, die von einem Kapillargefäße umfassenden Gewebe abgeleitet ist.

6. Zellpopulation nach einem der Ansprüche 1 bis 5, welche die Fähigkeit zur Kugelbildung aufweist.

7. Zellpopulation nach einem der Ansprüche 1 bis 6, die sich in mesenchymale Zellen differenzieren lässt.

8. Zellpopulation nach einem der Ansprüche 1 bis 6, die sich in neurale Zellen differenzieren lässt.

9. Zellpopulation nach einem der Ansprüche 1 bis 8, welche ein gefäßbildendes Potential aufweist.

10. Medizinisches Material zur Gewebe- oder Organregeneration, umfassend die Zellpopulation nach einem der Ansprüche 1 bis 9.

11. Medizinisches Material nach Anspruch 10, wobei ein Gewebe oder ein Organ aus mesenchymalen und neuralen Geweben oder Organen ausgewählt ist.

12. Verfahren zum Herstellen einer multipotenten Stammzellpopulation, umfassend den Schritt:
des Trennens adhärenter Zellen von den Zellen des Kapillargefäße umfassenden Gewebes;
des Trennens von Perizytenmarker positiven Zellen von den adhärenten Zellen; und
des Trennens einer EphA7-positiven Zelle von den Perizytenmarker positiven Zellen.

13. Verfahren nach Anspruch 12, wobei das Kapillargefäße umfassende Gewebe ein beliebiges Gewebe ist, das ausgewählt ist aus subkutanem Fettgewebe, Skelettmuskelgewebe, kardialem Gewebe, Nierengewebe und viszeralem Fettgewebe.

## Revendications

1. Population de cellules souches multipotentes humaines de type cellules souches mésenchymateuses isolées, qui est positive pour EphA7 et un ou plusieurs marqueurs de péricytes.

2. Population de cellules selon la revendication 1, qui est positive pour un ou plusieurs marqueurs de cellules souches neurales.

3. Population de cellules selon la revendication 1 ou 2, qui est négative pour CD34 et CD45.

4. Population de cellules selon l'une quelconque des revendications 1 à 3, qui n'est pas dérivée d'un cerveau.

5. Population de cellules selon l'une quelconque des revendications 1 à 4, qui est dérivée d'un tissu comprenant des vaisseaux capillaires.

6. Population de cellules selon l'une quelconque des revendications 1 à 5, ayant une capacité de formation de sphères.

7. Population de cellules selon l'une quelconque des revendications 1 à 6, qui peut se différencier en cellules mésenchymateuses.

8. Population de cellules selon l'une quelconque des revendications 1 à 6, qui peut se différencier en cellules neurales.

9. Population de cellules selon l'une quelconque des revendications 1 à 8, ayant un potentiel vasculogène.

10. Matériau médical pour une régénération de tissu ou d'organe, comprenant la population de cellules selon l'une quelconque des revendications 1 à 9.

11. Matériau médical selon la revendication 10, dans lequel un tissu ou un organe est choisi parmi des tissus ou organes mésenchymateux et neuraux.

12. Méthode pour la production d'une population de cellules souches multipotentes, comprenant les étapes de:
séparation de cellules adhérentes à partir des cellules du tissu comprenant des vaisseaux capillaires;
séparation de cellules positives aux marqueurs de péricytes à partir des cellules adhérentes; et
séparation d'une cellule positive à EphA7 à partir des cellules positives pour des marqueurs de péricytes.

13. Méthode selon la revendication 12, dans laquelle le tissu comprenant des vaisseaux capillaires est n'importe quel tissu choisi parmi un tissu adipeux sous-cutané, un tissu musculaire squelettique, un tissu cardiaque, un tissu rénal et un tissu adipeux de viscère.
